# EUROPEAN PATENT APPLICATION

(11) **EP 1 839 680 A1**
(43) Date of publication of application: **03.10.2007**
(21) Application number: 07251392.2
(22) Date of filing: 30.03.2007
(51) Int. Cl.: A61L 2/18, C11D 11/00, B08B 3/00, A01N 25/16, A01N 59/00, C11D 3/37, C11D 3/39, A61L 101/22

(54) **Composition for a foam pretreatment for medical instruments**

(30) Priority: 31.03.2006 US 396187; 30.11.2006 US 565126
(71) Applicant: ETHICON, INC., Somerville, New Jersey 08876 (US)
(72) Inventor: Lin, Szu-Min, Irvine, CA 92620 (US); Platt, Robert C., Laguna Niguel, CA 92677 (US); Tseng, Chun-Chieh J., Irvine, CA 92603 (US); Mosher, Robert F., Quail Hill, CA 92603 (US)
(74) Representative: Fisher, Adrian John

(57) **Abstract**

A foamable instrument pretreatment composition includes hydrogen peroxide in a range of from 0.1% to 15% by weight, a surfactant in a range of from 0.5 to 20% by weight; and a foam boosting agent comprising a silicone of from 0.1% to 10% by weight. It is intended for pretreating instruments after use in a medical procedure and before they are washed.

## Description

### BACKGROUND OF THE INVENTION

The present application relates to processing of medical instruments prior to reuse, and more particularly to pretreatment of the instruments prior to a sterilization process.

Medical instruments after use are typically contaminated with blood and other body matter as well as potentially contaminated with infectious microorganisms. Before being reused in a future medical procedure these instruments must be washed and sterilized. The process of washing and sterilization becomes complicated when blood and other matter are allowed to dry onto the instruments. Blood in particular becomes much more difficult to remove once it has dried.

It has been suggested that after use instruments be placed into a liquid filled container to maintain moisture and prevent foreign matter thereon from drying and becoming more difficult to remove. However, such containers can be quite heavy and difficult to move and the liquid therein can become contaminated and it is not desirable to spill this liquid. One solution that has been proposed is an enzymatic foam which is prayed onto instruments after use and prior to eventual sterilization. The foam weighs less than a liquid and purports to enhance cleaning by initiating some degree of cleaning at the early stage when the foam is placed upon the instrument. Such foams provide little or no antimicrobial activity.

### SUMMARY OF THE INVENTION

A foamable instrument pretreatment composition according to the present invention comprises hydrogen peroxide, a surfactant and a foam boosting agent comprising a silicone. Preferably, the hydrogen peroxide is present in a range of from 0.1% to 15% by weight, more preferably in a range from about 2% to 10% by weight, and most preferably in a range from about 3% to 8% by weight. Preferably, the surfactant is present in a range of from 0.5 to 20% by weight, more preferably in a range from about 1% to 10% by weight, and most preferably in a range from about 2% to 6% by weight. Preferably, the foam boosting agent is present in a range of from 0.1% to 10% by weight, more preferably in a range from about 0.3% to 5% by weight, and most preferably in a range from about 0.5% to 3% by weight.

Preferably, the pH is in the range of from 4.7 to 7.5, more preferably in a range of from 5 to 7, and most preferably in a range of from 5.5 to 6.5. Preferably, the composition further comprises a thickening agent comprising an acrylic polymer in an amount of from about 0.5% to 20%, more preferably from about 1% to 10% and most preferably from about 1.5% to 5%.

The composition can be packaged in a pressurized foam dispensing container. It can also be packaged in a manually pumped foam dispensing container. It can also further include peracetic acid.

### BRIEF DESCRIPTION OF THE DRAWINGS

FIG. 1 is a block diagram of a system according to the present invention;
FIG. 2 is a block diagram of an enhanced system of FIG. 1;
FIG. 3 is a front elevation view of a foam dispenser for use in the system of FIG. 1;
FIG. 4 is a front elevation view of an alternative foam dispenser for use in the system of FIG. 1;
FIG. 5 is a front elevation view in cross-section of a container for use in the system of FIG. 1;
FIG. 6 is a front elevation view in cross-section of an alternative container for use in the system of FIG. 1;
FIG. 7 is a front elevation view in cross-section of a further alternative container for use in the system of FIG. 1; and
FIG. 8 is a front elevation view in cross-section of a multi-component foam dispenser for use in the system of FIG. 1.

### DETAILED DESCRIPTION

During a medical procedure, one or more medical instruments may be employed. These instruments become contaminated with blood, tissue and potentially contaminating microorganisms. Typically the instruments are set aside after use to await washing and sterilization. This waiting period can be several hours or much longer. During this waiting period blood and other matter which dries upon the instrument becomes much more difficult to remove during the subsequent cleaning procedure. This can be a particular problem when a procedure lasts many hours and uses many different instruments or when due to limited personnel time, it is difficult to process the instruments in a timely fashion.

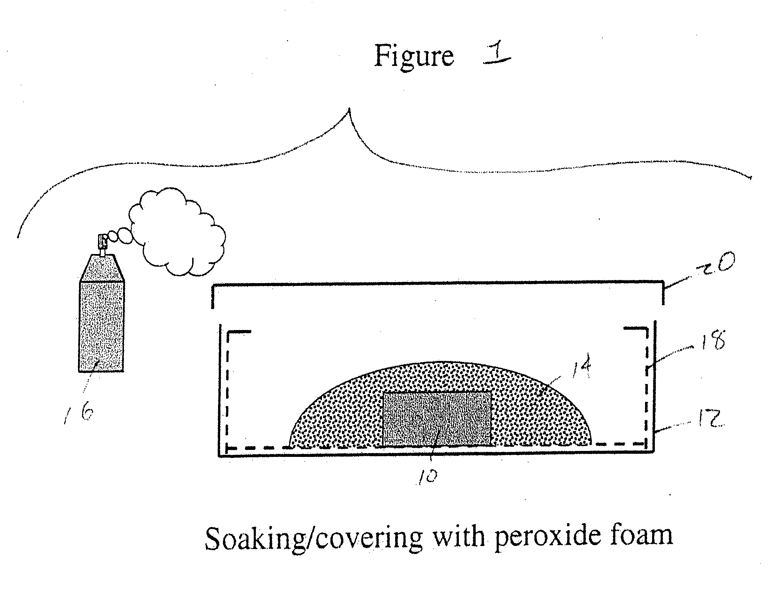

Turning to the drawings, and in particular to FIG. 1, according to the present invention, after use and prior to a complete washing and sterilization procedure the instruments 10 are placed into a container 12 and covered with a foam 14. The foam comprises hydrogen peroxide. The hydrogen peroxide foam 14 acts to dissolve blood, even dried on blood, and to initiate antimicrobial activity against microorganisms on the instrument. The foam 14 encapsulates the instruments 10 and maintains a moist state thereon to inhibit drying of blood and other matter on the instrument. Keeping the blood and other matter from drying promotes superior washing in a subsequent washing and sterilization process.

One method of dispensing the hydrogen peroxide foam 14 would be to spray the foam 14 from a foaming aerosol spray can 16. Such cans employing a propellant are well known to those of skill in the art. Also, the container 12 preferably includes an insert or tray 18 having a plurality of apertures therethrough to allow easy rinsing of the instruments 10 and for efficient diffusion of vapor sterilants into contact with the instruments 10 when the container 12 is used in a sterilization procedure. A lid 20 is also preferably provided.

Instruments 10 are placed into the container 12 as they are finished being used in a procedure. A quantity of foam 14 is sprayed over the instruments 10 to keep them moist and inhibit drying of blood thereon, to start dissolving the blood thereon and to disinfect the instruments. The foam 14 preferably contains between 1 to 15 percent hydrogen peroxide by weight and more preferably between about 3 to 8 percent. Such concentration may not achieve a level of sterilization sufficient for immediate reuse on a patient, but will substantially reduce the load of microorganisms on the instrument surfaces so as to minimize the chances that personal handling the instruments, especially during cleaning, will get infected from them. The lid 20 is preferably placed on the container 12 prior to transporting the instruments from the location of the procedure, such as an operating room, to the location of the washing. When the instruments 10 are ready for washing, the insert 18 can be lifted out and the foam 14 rinsed off while the instruments 10 are still in the insert 18. Normal washing and sterilization may then occur. Washing may comprise treatment with enzymatic cleansers, detergents or other cleaning agents, preferably in combination with mechanical scrubbing or agitation, including optionally treatment with water jets, ultrasonic vibration or the like. Following washing the instrument should be sterilized, preferably in the container 12, such as by chemical vapor or steam autoclaving.

It is particularly convenient if the container 12 with the insert 18 is adapted for use in the terminal sterilization such as a STERRAD^{®} hydrogen peroxide / gas plasma system or a steam system. Suitable materials, such as liquid crystal polymers, and construction details for such containers, especially containers adaptable to either steam or hydrogen peroxide, are shown in US Patent Nos. 6,379,631 and 6, 692, 693 to Wu incorporated herein by reference. Such containers are typically wrapped with CSR wrap or incorporate semi-permeable membrane filters to allow sterilization of instruments therein with vapor sterilants while protecting the against ingress of potentially contaminating microorganisms after sterilization.

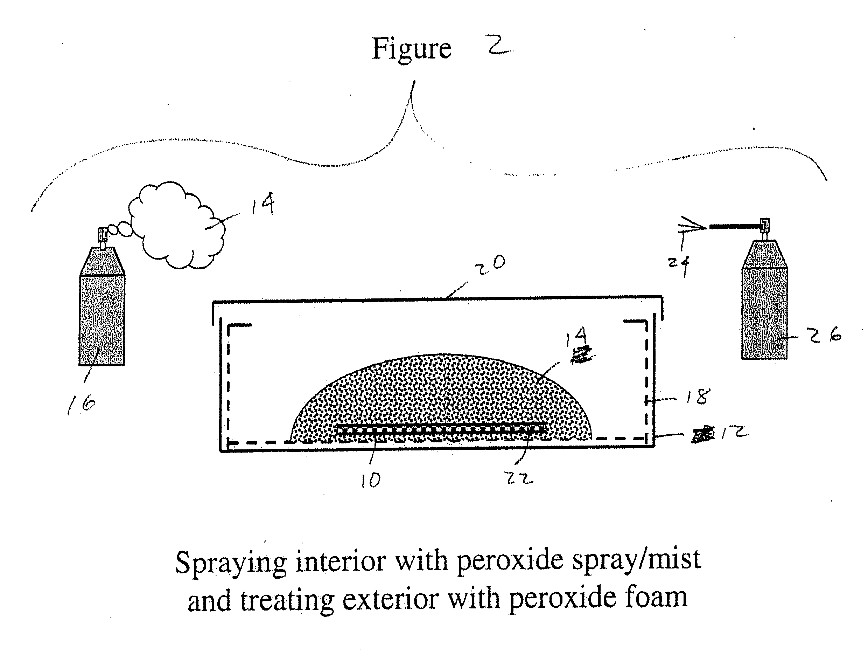

Turning also now to FIG. 2, in addition to covering an exterior surface of the instrument 10 with the hydrogen peroxide foam 14, if the instrument 10 has a lumen 22, a liquid or mist 24 comprising hydrogen peroxide is preferably sprayed into the lumen 22 prior to placing the instrument 10 into the container 12 and covering the instrument 10 with foam 14. The mist is also preferably dispensed from a pressurized container 26 employing a propellant as is known in the art.

Turning also now to FIG. 3, to enhance convenience, a dispenser 28 can be provided with a foaming nozzle 30 and misting nozzle 32. A foamable hydrogen peroxide solution and a propellant are in the dispenser 28 and when distributed through the misting nozzle 32 the solution comes out as a mist 34 appropriate for squirting into a lumen and when dispensed through the foaming nozzle 30 the solution comes out as a foam 36 appropriate for covering exterior surfaces of an instrument.

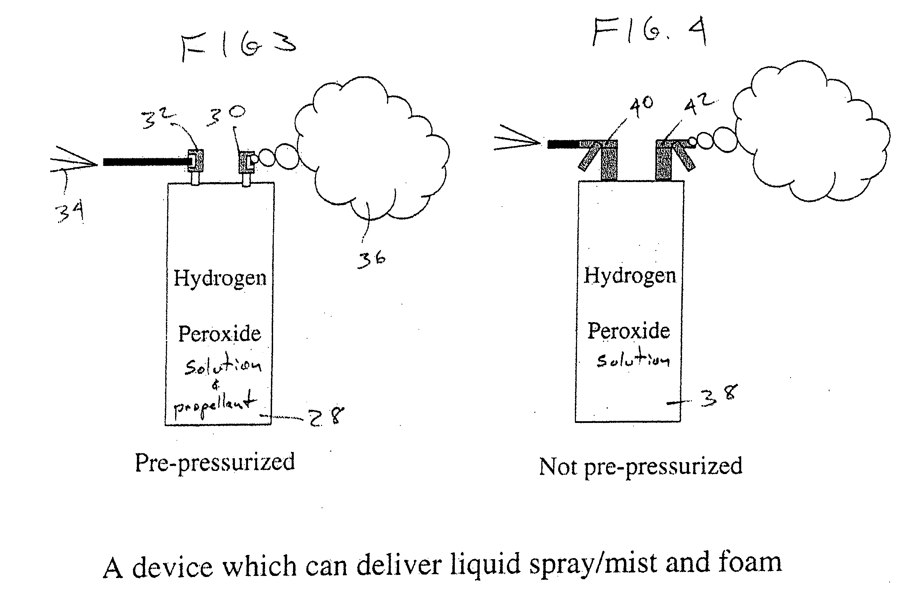

Turning also now to FIG.4, rather than employ a propellant, a dispenser 38 having a foamable solution of hydrogen peroxide therein may employ manually operated misting nozzle 40 and foaming nozzle 42. A particularly useful foaming nozzle 42 is the Airspray F2-L11 available from Airspray NV, Alkamar, The Netherlands.

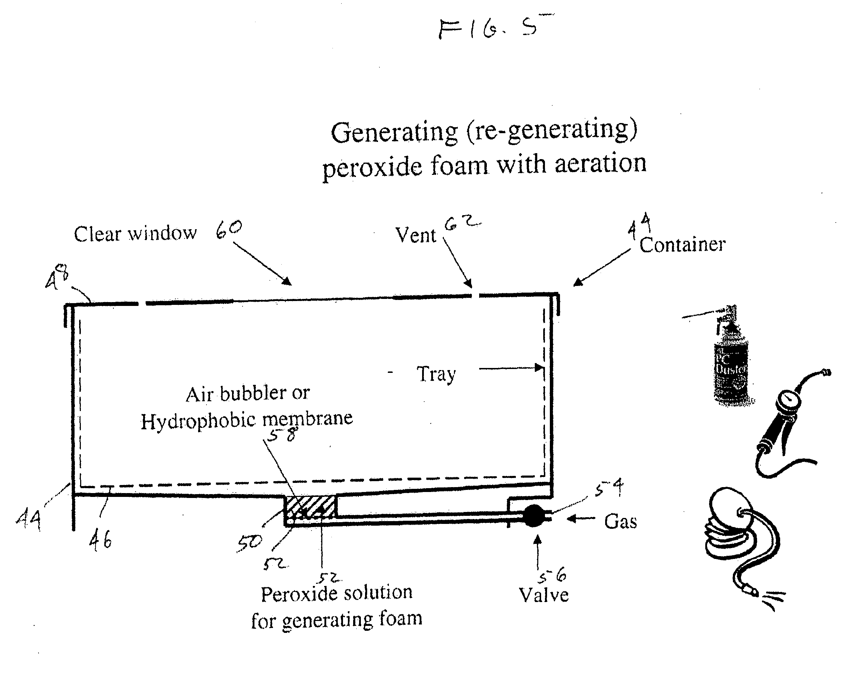

Turning also now to FIG. 5, a container 44 is illustrated having a mesh insert 46 and lid 48. A lower portion of the container has a well 50 into which a quantity of foamable hydrogen peroxide solution 52 may be placed. A port 54 and valve 56 connect to the well 50 through an air bubbler or hydrophobic membrane 58. A supply of compressed air or other gas attached to the port 54 percolates through the bubbler 58 to foam the hydrogen peroxide solution 52 and fill the container 44 with the hydrogen peroxide foam. Preferably, the lid 48 contains a viewing window 60 to view the progress of foam filling the container 44 and one or more vents 62 to allow gases in the container 44 to escape and allow the foam to fill the container 44. The vent 62 may be a simple opening, or be covered with a semi-permeable membrane or employ a one-way valve.

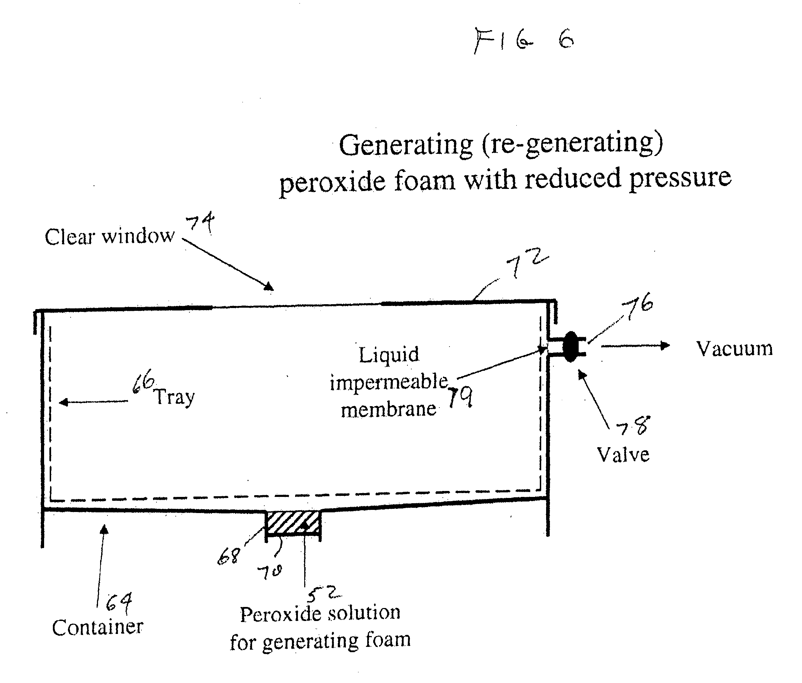

Turning also to FIG. 6, an alternative container 64 as structured similarly to the container 44 with an insert 66 well 68 with a hydrophobic membrane 70 and a lid 72 with a window 74 rather than a port for compressed air or gas, a port 76 is provided on an upper location of the container 64 and has a valve 78 and an additional hydrophobic membrane 79. By attaching the port 76 to a source of vacuum and drawing gases out of the container 64, air will percolate into the container through the hydrophobic membrane 70 providing a foaming action to hydrogen peroxide solution 52 in the well 68. In either this container 64 or the previous container 44, if the foam dissipates, it can be refoamed by employing the vacuum or compressed gas as the case may be.

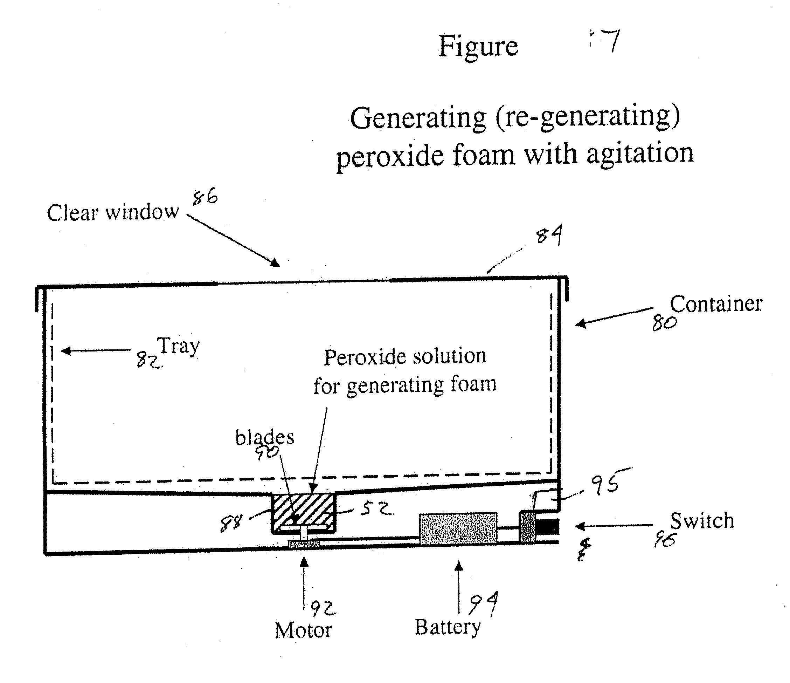

Turning also now to FIG. 7, a container 80 having an insert 82 and lid 84 with a window 86 has a well 88. An agitator 90 sits within the well 88 and is attached to a motor 92 and power source, such as a battery 94, which is controlled via a switch 96. Engaging the agitator 90 foams a hydrogen peroxide solution 52 in the well 88 to fill the container 80. Any of the foam generating schemes can also benefit from a timer 95 which can automatically engage the foam generating apparatus such as the agitator 90 to reconstitute the foam at predetermined intervals thereby ensuring good contact with the instruments even during extended periods.

### Examples

### Formulation 1

| **Type of foam** | **Mousse-Like Thick Foams** |
|---|---|
| Application | Spray |
| Ingredients | Wt (g) |
| Deionized Water | 60.0 |
| Carbopol Aqua SF-1 Polymer | 3.4 |
| Tween 80 | 2.0 |
| Glycerol | 2.0 |
| NaOH (1.0N) | As needed |
| H₂O₂ | As needed |
| Preservative(s) | As needed |

### Formulation 2

| **Type of foam** | **Mousse-Like Thick Foams** |
|---|---|
| Application | Spray |
| Ingredients | Wt (g) |
| Deionized Water | 120.0 |
| Carbopol Aqua SF-1 Polymer | 6.8 |
| Tween 80 | 4.0 |
| Glycerol | 1.0 |
| NaOH (1.0N) | As needed |
| H₂O₂ | As needed |
| Preservative(s) | As needed |

### Formulation 3

| **Type of foam** | **High Foaming** |
|---|---|
| Application | Aeration/Vacuum/Spray |
| Ingredients | Wt (g) |
| Deionized Water | 78.0 |
| Fixate G-100 Polymer | 6.0 |
| Tween 80 | 1.0 |
| SilSense Copolyol-1 Silicone | 1.0 |
| Glycerin | 4.0 |
| H₂O₂ | As needed |
| Preservative(s) | As needed |

### Formulation 4

| **Type of foam** | **High Foaming** |
|---|---|
| Application | Aeration/Vacuum/Spray |
| Ingredients | Wt (g) |
| Deionized Water | 85.0 |
| SilSense Q-Plus Silicone | 1.0 |
| Tween 80 | 2.0 |
| Glycerol | 3.0 |
| 59% H₂O₂ | 5.0 |
| Preservative(s) | As needed |

### Formulation 5

| **Type of foam** | **High Foaming** |
|---|---|
| Application | Aeration/Vacuum/Spray |
| Ingredients | Wt (g) |
| Deionized Water | 91.0 |
| Fixate G-100 Polymer | 6.0 |
| Tween 80 | 1.0 |
| SilSense Q-Plus Silicone | 1.0 |
| 59% H₂O₂ | 5.0 |
| Preservative(s) | As needed |

### Formulation 6 (for ~ 6% peroxide)

| **Type of foam** | **High Foaming** |
|---|---|
| Application | Aeration/Vacuum/Spray |
| Ingredients | Wt (g) |
| Deionized Water | 150.0 |
| Tween 80 | 8.0 |
| SilSense Copolyol-1 Silicone | 2.0 |
| 59% H₂O₂ | 18.0 |

### Formulation 7 (for - 3% peroxide)

| **Type of foam** | **High Foaming** |
|---|---|
| Application | Aeration/Vacuum/Spray |
| Ingredients | Wt (g) |
| Deionized Water | 150.0 |
| Tween 80 | 8.0 |
| SilSense Copolyol-1 Silicone | 2.0 |
| 59% H₂O₂ | 9.0 |

### Formulation 8 (Defoaming and neutralizing solution)

| De-foaming agent (Rug Doctor water-based silicone emulsion) | **1%** |
|---|---|
| Catalase | ~1000 units/ml |
| Water | Remainder |

Preferably, a defoaming solution such as Formulation 8 is provided in a spray dispenser, either manually pumped or with a propellant gas and is provided with instructions for defoaming a hydrogen peroxide foam 14 which covers instruments 10. Prior to defoaming the instruments 10 are covered by the hydrogen peroxide foam 14 making it difficult for a user to see the instruments 10 and remove them from the container 12. If the instruments 10 have sharp points or edges the user might risk injury by reaching into the foam 14 without being able to adequately see the instruments 10. Preferably, the defoaming solution comprises both a defoaming agent and agent for inactivating the hydrogen peroxide. Thus, upon spraying the defoaming solution over the foam 14 the foam's volume is reduced so that the instruments can be seen for safe removal and the concentration of hydrogen peroxide in the foam 14 is reduced to minimize any detrimental effects its contact with a user might present.

The two most common types of defoamers are organic-based defoamers such as polypropylene based polyether dispersions (Sigma antifoam 204) and fatty acid esters (Sigma antifoam O-30), and silicone-based defoamers such as siloxane polymers (Sigma antifoams A, B, C, Y-30, SE-15). Silicone based defoamers are somewhat preferred due to the ease with which they can be cleaned from an instrument 10 compared with organic based defoamers. However, either type may be used. One appropriate defoaming agent is SILSENSE Copolyol-1 silicone which is a polyethylene glycol (organic) and dimethicone (silicone) copolyol. Additional appropriate defoaming agents include: carboxylates (organic based), monoamides (organic based), phosphoric acid esters (organic based), mineral oil blends (organic based), long chain alcohols (organic based), fluorosurfactants (organic based), hydrophobed silicon/hydrophilic oil mixtures (silicone based), Silicas (such as polydimethylsiloxane polymer with silica) (silicone based), diethylene glycol (organic based), polydiethylenemethyl silicones (silicone based).

For neutralizing hydrogen peroxide in the foam 14 catalase is preferred due to its ease of use, potent action against hydrogen peroxide, ease of removal and low toxicity. Other agents include cobalt salts, idodide salts, titanium salts, ceric salts and permanganate salts.

### Formulation 9 (Foaming Mousse (3% H₂O₂))

| Ingredient | Amount (g) | Weight % | Function | Material Type |
|---|---|---|---|---|
| Deionized Water | 120 | 83.3 | Solvent | Aqueous Phase |
| Carbopol AQUA SF-1 (35%) | 10 | 6.9 | Thickener | Acrylic Polymer |
| Tween 80 | 4 | 2.8 | Foaming Agent | Surfactant |
| SilSense Q-Plus Silicone | 1 | 0.7 | Foam Booster Tack Reducer | Modified Silicone Liquid |
| Hydrogen Peroxide (59%) | 9 | 6.3 | Disinfecting agent Decontaminating agent | Oxidizer |
| Sodium Hydroxide (0.1N) | As needed | <1.0 | pH Modifier | Basic solution |
| Citric Acid (50%) | As needed | <1.0 | pH Modifier | Acidic solution |

| | | | | |
|---|---|---|---|---|
| Final pH= 6.1 | | | | |

### Modified formulation 7 (with pH adjustor)

| High-Foaming (3% H₂O₂) | | | | |
|---|---|---|---|---|
| Ingredient | Amount (g) | Weight % | Function | Material Type |
| Deionized Water | 150 | 88.8 | Solvent | Aqueous Phase |
| Tween 80 | 8 | 4.7 | Foaming Agent | Surfactant |
| SilSense Copolyol-1 Silicone | 2 | 1.2 | Foam Booster Tack Reducer | Modified Silicone Liquid |
| Hydrogen Peroxide (59%) | 9 | 5.3 | Disinfecting agent Decontaminating agent | Oxidizer |
| Sodium Hydroxide (0.1N) | As needed | < 1.0 | pH Modifier | Basic solution |
| Citric Acid (50%) | As needed | < 1.0 | pH Modifier | Acidic solution |

| | | | | |
|---|---|---|---|---|
| Final pH= 6.0 | | | | |

### Modified formulation 6 (with pH adjustor)

| Hi-Foaming. (6% H₂O₂) | | | | |
|---|---|---|---|---|
| Ingredient | Amount (g) | Weight % | Function | Material Type |
| Deionized Water | 150 | 84.3 | Solvent | Aqueous Phase |
| Tween 80 | 8 | 4.5 | Foaming Agent | Surfactant |
| SilSense Copolyol-1 Silicone | 2 | 1.1 | Foam Booster Tack Reducer | Modified Silicone Liquid |
| Hydrogen Peroxide (59%) | 18 | 10.1 | Disinfecting agent Decontaminating agent | Oxidizer |
| Sodium Hydroxide (0.1N) | As needed | < 1.0 | pH Modifier | Basic solution |
| Citric Acid (50%) | As needed | <1.0 | pH Modifier | Acidic solution |

| | | | | |
|---|---|---|---|---|
| Final pH= 5.6 | | | | |

### Preferred formulation

| | Preferred | More preferred | Most Preferred |
|---|---|---|---|
| Hydrogen peroxide | 0.1 - 15% | 2 - 10% | 3 - 8% |
| Surfactant | 0.5 - 20% | 1 - 10% | 2 - 6% |
| Foam booster (Modified silicone) | 0.1 - 10% | 0.3 - 5% | 0.5 - 3% |
| Thickening agent (Acrylic polymer) | 0.5 - 20% | 1 - 10% | 1.5 - 5% |
| pH | 4.5 - 7.5 | 5 - 7 | 5.5 - 6.5 |

### Tests

### (A) Test with fresh blood

A drop of fresh blood, approximately four millimeters in diameter was applied to a Petri dish. One was left untreated and the other treated with a peroxide foam of formulation 7 generated with Airspray F2-L11 Finger Pump Foamer. Within ten minutes the untreated blood had dried whereas the treated blood had reacted and dissolved in the peroxide foam.

### (B) Tests with dried blood

A drop of dried blood was treated with room temperature tap water for ten minutes and another drop of dried blood was treated with a 3% hydrogen peroxide foam of formulation 7 generated with Airspray F2-L11 Finger Pump Foamer. The drop of dried blood treated with tap water remained after ten minutes. After ten minutes, the drop of dried blood treated with the hydrogen peroxide foam had dissolved.

An additional test was conducted comparing a commercially available enzyme foam, Prepzyme XF enzyme foam, available from Ruhof Corporation of Mineola, NY. A drop of dried blood was treated with the Prepzyme XF and another drop of dried blood was treated with a 6% hydrogen peroxide foam of formulation 6. After ten minutes the blood treated with the Prepzyme XF remained whereas the blood treated with the hydrogen peroxide foam was dissolved within five minutes.

### (C) Foam stability test

A foam prepared according to formulation 9 was placed into a Petri dish of dimensions 150 mm diameter and 15 mm deep. Prepzyme XF was placed into a similar Petri dish. The foams were allowed to rest for one hour whereupon they were inspected. The foam of formulation 9 maintained substantially all of its volume over the period of one hour. The Prepzyme foam had fallen to the extent that a portion of the lower surface of the Petri dish was no longer covered by foam. After four hours the foam of formulation 9 still covered the bottom surface of the Petri dish.

### (D) Tests against microorganisms

Tests of efficacy in killing microorganisms were conducted comparing both a 3% hydrogen peroxide foam prepared according to formulation 7 and 6% hydrogen peroxide foam prepared according to formulation 6 against the Prepzyme XF enzymatic foam using the following test procedure:
Step 1: Place microorganism suspension onto sterile filter
Step 2: Allow the suspension to dry
Step 3: Add either peroxide foam or enzyme foam to cover filter
Step 4: Allow foam to set on microorganism for pre-determined time
Step 5: Rinse filter with 10 mL sterile neutralizing/defoaming solution (formulation 8)
Step 6: Rinse filter with three times of 100 mL sterile water
Step 7: Place filter on TSA agar and incubate @ 32C for 48hours
Step 8: Determine the number of survivors (TNTC = Too Numerous to Count)

### Efficacy results with duplicated samples:

| | | |
|---|---|---|
| Control | Staphylococcus Aureus | Pseudomonas aeruginosa |
| | TNTC & TNTC (Average: 1.64x10⁵) | TNTC & TNTC (Average: 2.49x10⁵) |

| Exposure Time (Minutes) | Foam | Staphylococcus aureus | Pseudomonas aeruginosa |
|---|---|---|---|
| 5 | No foam with catalase/de -foaming agent (Control) | TNTC & TNTC | TNTC & TNTC |
| | Enzyme foam (Ruhof Prepzyme XF) | TNTC & TNTC | TNTC & TNTC |
| | 3% hydrogen peroxide foam | TNTC & TNTC | 16 & 37 |
| | 6% hydrogen peroxide foam | ~500 & ~500 | 0 & 0 |
| 10 | Enzyme foam (Ruhof Prepzyme XF) | TNTC & TNTC | TNTC & TNTC |
| | 3% hydrogen peroxide foam | ~1000 & ~1000 | 0 & 1 |
| | 6% hydrogen peroxide foam | 46 & 22 | 0 & 0 |

The hydrogen peroxide foams of the present invention will be quite effective against blood borne pathogens such as hepatitis and HIV. Accordingly, treatment of the instruments with the foam can help prevent accidental infections of medical personnel who come into contact with the instruments.

Additional supplements may be desirable in the foam 14. For instance, to protect the instruments 10 from corrosion it may be desirable to add a corrosion inhibitor. U.S. Pat. No. 6,585,933 entitled "Method and composition for inhibiting corrosion in aqueous systems," the entire contents of which are incorporated herein by reference, provides many good corrosion inhibitor references and examples. A corrosion inhibitor is a chemical compound that stops or slows down corrosion of metals and alloys. Some of the mechanisms of its effect are formation of a passivation layer, inhibiting either the oxidation or reduction part of the redox corrosion system, or scavenging the dissolved oxygen. Some corrosion inhibitors are triazoles (benzotriazole, hydrobenzotriazole, carboxybenzotriazole), azoles, molybdates (sodium molybdate), vanadates, sodium gluconate, benzoates (sodium benzoate), tungstates, azimidobenzene, benzene amide, zinc oxide, hexamine, phenylenediamine, dimethylethanolamine, sodium nitrite, cinnamaldehyde, condensation products of aldehydes and amines (imines), alkanolamides, chromates, dichromates, borates, nitrites, phosphates, hydrazine, ascorbic acid, sodium silicate, sodium resinate and combination thereof. The preferred corrosion inhibitors include alkanolamide, sodium silicate, and triazoles. Preferably, the concentration of corrosion inhibitor is from about 0.01 to about 10% by weight, more preferably from about 0.05 to about 2%, and most preferably from 0.1 to about 1.5%.

Lipid, often used as a synonym for fat, is a class of hydrocarbon-containing organic compound. Lipids are soluble in non-polar solvent and are relatively insoluble in water. The biological contaminants on the instrument will usually include lipids and it thus may be desirable to include a lipid solvent in the foam 14 to hasten the breakdown and removal of these lipids.

Some lipid solvents include alcohols (methanol, ethanol, and 1-propanol), ethers (diethyl ether and petroleum ether), glycol ethers (propylene glycol t-butyl ether and dipropylene glycol methyl ether), acetone, carbon tetrachloride, chloroform and citrus-based solutions containing d-limonene. For the purpose of this foam 14, dislodging lipid/fat from the surfaces of medical devices during the treatment may be sufficient. The complete dissolving of lipid/fat may not be needed. The preferred lipid solvents include glycol ethers and d-limonene. Preferably, the concentration of solvent to dislodge lipid/fat is from about 0.01 to about 10% by weight, more preferably from about 0.1 to about 5%, and most preferably from 1 to about 3%.

It has been shown that a combination of hydrogen peroxide with acetic acid provides an enhanced effect in sterilization over hydrogen peroxide alone. Preferably, they are dispensed from separate adjacent containers. If from a spray, as for lumens, separate sprayers remain workable. However, for foam it is desirable to have a consistent mixture of the two in a single foam. Accordingly, it would be desirable to simultaneously dispense both the hydrogen peroxide containing foam 14 and a foam containing the acetic acid. The DUAL FOAMER available from Airspray International Inc., Pompano Beach, Florida, dispenses side by side foams from separate yet adjoining containers.

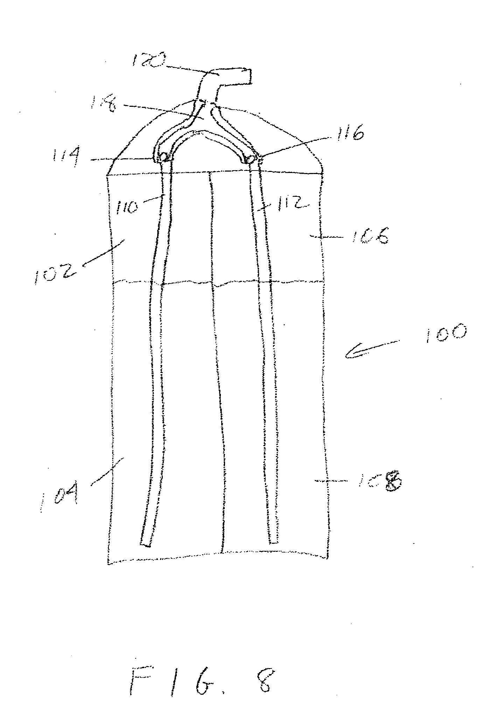

Even better would be to mix the two ingredients just prior to foaming. FIG. 8 shows a foam dispensing container 100 having a first compartment 102 containing a hydrogen peroxide solution 104 suitable for foaming as herein described and a second compartment 106 containing an acetic acid solution 108 suitable for foaming. The acetic acid solution 108 is similar to the hydrogen peroxide solution with the substitution of acetic acid for hydrogen peroxide in the formulation (giving the solution an amount of acetic acid of 5% by weight). Tubes 110 and 112, having one-way valves 114 and 116 respectively, lead from the first compartment 102 and second compartment 106 respectively to a mixing chamber 118 to which is connected a foam dispensing nozzle 120. A propellant in the first compartment 102 and second compartment 106 provides the motive force.

An alternative hand operated component mixing foam dispenser is shown in US Patent No. 5,918,771 entitled "Aerosol Intended for Dispensing a Multi-Component Material," the entire contents of which are incorporated herein by reference. An alternative propellant driven binary component mixing foam dispenser is disclosed in US Patent No. 6,305,578, the entire contents of which are incorporated herein by reference.

Battery powered sprayers may also be convenient. The BOS-2 sprayer available from Saint-Gobain Calmar, Inc. of Grandview, MO is one such option. It incorporates a battery operated pumping head and a variety of available spray nozzles including one which converts from foam to spray to closed via a twist on the outlet portion. Such a sprayer can provide a large volume of foam without large volumes of propellant and without using undo manual actuation.

In practice, instruments 10 are placed into the container 12 as they are finished being used during a medical procedure. A small amount of hydrogen peroxide foam 14 can be added over each instrument 10 as it is placed in the container 12 if there will be lengths of time in between placing instruments therein. A user may wait until all instruments 10 are in the container to apply the foam 14 and cover the instruments 10 therewith and then place the cover on the container. The foam 14 is light allowing easy transport of the container 12 with instruments 10 and foam 14 therein from the site of the procedure to the site where terminal decontamination and sterilization will occur.

When a user is ready to process the instruments the cover is removed and the defoaming solution is sprayed onto the foam 14 covering the instruments 10. The defoaming agent in the solution breaks down the physical structure of the foam 14 and the deactivating agent breaks down the hydrogen peroxide, preferably into water and oxygen. If desired, they may be rinsed in the container with fresh water or other solvent. The user then processes the instruments in their usual manner.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof.

## Claims

1. A foamable instrument pretreatment composition comprising:
hydrogen peroxide in a range of from 0.1% to 15% by weight;
a surfactant in a range of from 0.5 to 20% by weight; and
a foam boosting agent comprising a silicone of from 0.1% to 10% by weight.

2. A foamable instrument pretreatment composition according to claim 1 wherein the hydrogen peroxide is present in a range from about 2% to 10% by weight.

3. A foamable instrument pretreatment composition according to claim 2 wherein the hydrogen peroxide is present in a range from about 3% to 8% by weight.

4. A foamable instrument pretreatment composition according to any of claims 1, 2 and 3 wherein the surfactant is present in a range from about 1% to 10% by weight.

5. A foamable instrument pretreatment composition according to any of claims 1, 2 and 3 wherein the surfactant is present in a range from about 2% to 6% by weight.

6. A foamable instrument pretreatment composition according to any of claims 1, 2, 3, 4 and 5 wherein the foam boosting agent is present in a range from about 0.3% to 5% by weight.

7. A foamable instrument pretreatment composition according to any of claims 1, 2, 3, 4 and 5 wherein the foam boosting agent is present in a range from about 0.5% to 3% by weight.

8. A foamable instrument pretreatment composition according to any of claims 1 to 7 wherein the pH is in a range of from 4.7 to 7.5.

9. A foamable instrument pretreatment composition according to any of claims 1 to 7 wherein the pH is in a range of from 5 to 7.

10. A foamable instrument pretreatment composition according to any of claims 1 to 7 wherein the pH is in a range of from 5.5 to 6.5.

11. A foamable instrument pretreatment composition according to any of claims 1 to 10 and further comprising a thickening agent comprising an acrylic polymer in an amount of from about 0.5% to 20% by weight.

12. A foamable instrument pretreatment composition according to any of claims 1 to 10 and further comprising a thickening agent comprising an acrylic polymer in an amount of from about 1% to 10% by weight.

13. A foamable instrument pretreatment composition according to any of claims 1 to 10 and further comprising a thickening agent comprising an acrylic polymer in an amount of from about 1.5% to 5% by weight.

14. A foamable instrument pretreatment composition according to any of claims 1 to 13 packaged in a pressurized foam dispensing container.

15. A foamable instrument pretreatment composition according to any of claims 1 to 13 packaged in a manually pumped foam dispensing container.

16. A foamable instrument pretreatment composition according to any of claims 1 to 15 and further comprising peracetic acid.

17. A foamable instrument pretreatment composition according to any of claims 1 to 16 and further comprising a corrosion inhibitor.

18. A foamable instrument pretreatment composition according to claim 17 wherein the corrosion inhibitor is selected from the group of alkanolamide, sodium silicate, and triazoles.

19. A foamable instrument pretreatment composition according to claim 17 or claim 18 wherein the corrosion inhibitor is present in the foam in an amount from about 0.01 to about 10% by weight.

20. A foamable instrument pretreatment composition according to claim 17 or claim 18 wherein the corrosion inhibitor is present in the foam in an amount from about 0.05 to about 2% by weight.

21. A foamable instrument pretreatment composition according to claim 17 or claim 18 wherein the corrosion inhibitor is present in the foam in an amount from 0.1 to about 1.5% by weight.
